# EUROPEAN PATENT SPECIFICATION

(11) **EP 3 004 441 B1**
(45) Date of publication and mention of the grant of the patent: **03.05.2017**
(21) Application number: 14735736.2
(22) Date of filing: 28.05.2014
(51) Int. Cl.: D04C 3/00, A61F 2/00

(54) **BRAIDING MACHINE FOR PRODUCING THREE-DIMENSIONAL BRAIDED MATRICES**
FLECHTMASCHINE ZUR HERSTELLUNG VON DREIDIMENSIONALEN GEFLOCHTENEN MATRIZEN
MACHINE À TRESSER POUR PRODUIRE DES MATRICES TRESSÉES EN TROIS DIMENSIONS

(30) Priority: 31.05.2013 US 201361829872 P
(43) Date of publication of application: 13.04.2016
(73) Proprietor: Soft Tissue Regeneration, Inc., New Haven, CT 06510 (US)
(72) Inventor: DUKE, Patrick W., Carolina Beach, North Carolina 28428 (US); REILLY, Joseph W., Chatham, New Jersey 07928 (US)
(74) Representative: Potter Clarkson LLP
(86) International application number: PCT/US2014/039740
(87) International publication number: WO 2014/193926

(56) References cited:
- EP-A2- 0 341 434
- WO-A1-2011/119831
- US-A- 5 392 683

## Description

### CROSS REFERENCE TO RELATED APPLICATION

This application claims priority to U.S. provisional application serial no. 61/829,872, filed May 31, 2013, the disclosure of which is incorporated herein in its entirety.

### FIELD OF THE INVENTION

The present invention relates to three-dimensional braiding machines and methods of manufacturing three-dimensional braided matrices.

### BACKGROUND OF THE INVENTION

A braided structure is typically manufactured using a system of equipment including a braiding machine, a forming device, including a forming ring, and a take-up device. Standard, maypole braiding machines contain a track plate, onto which a plurality of yarn carriers is positioned. The yarn carriers have a substantially perpendicular spindle and carry the spools of yarn or bobbins wound with yarn or yarn packages. The yarn carriers may use tension controls to release the yarn during processing.

Half of the yarn carriers are driven in a clockwise direction and half are driven in a counterclockwise direction. The movement of carriers is guided by the track plate that causes the two sets of opposing carriers to travel in a Maypole fashion around carrying yarns that extend perpendicular to the plane of the braiding machines track plate. At the point where the yarns consolidate to form the braid (frequently referred to as the braid point), a forming device is often used to control the dimension and shape of the braided fabric. Traditionally, the forming device comprises a forming ring that controls the outside diameter of the finished braided product. The tension required to pull the yarn off of the carriers and to pull the finished braid is supplied by a take-up device. The take-up device applies the force by pulling on the finished braid.

The path of the carriers is diverted by gears so that they pass either inside or outside others travelling in the opposite direction. The yarns carried by the moving carriers are wound under and over each other to produce a braid.

In operation, yarn is supplied from each carrier to a "braid point" where the braid is produced and drawn away. The speed at which the braid is drawn away or "taken up" may be varied to produce braids of differing structure e.g. "open" and "closed" braids.

To obtain satisfactory results, it is important for the rate at which the yarn is supplied to the braid point to be controlled. For example, to achieve a braid of even formation, it is important to ensure that the yarn is taken up from each bobbin at the same speed such that the same length of yarn is supplied from each bobbin. The yarn from each bobbin must also be sufficiently taut. In standard braiding machines, control is facilitated by the use of yarn take off guides and tensioning devices on each carrier.

Generally yarn from a bobbin or package mounted on a carrier passes through a first yarn guide, through a yarn guide of a tensioning device, and then through a second yarn guide before delivery to the braid point. In each case the guide might be a hook, roller, loop, aperture or other suitable engagement means.

With maypole type braiders, the maximum speed of rotation is severely limited by the need to continuously and repetitively change the path of movement of the carriers and the bobbins thereon. The inertia forces of the combined mass of the carriers and the bobbins, particularly when the strands are metal, is considerable and the vibration and wear caused by changing the direction of movement thereof is a severe limitation on the maximum speed of braiding. In the alternative, the bobbins can be made quite small but in such instances the length of the strands on each carrier is severely limited making it necessary to frequently stop the operation of the machine to replenish the machine with newly filled bobbins.

Another type of braiding machine is known as a rotary braiding machine. In these machines, there is a set of inner carriers, a set of outer carriers and a set of strand deflectors located between the inner and outer carriers. The inner and outer carriers follow a circular path about the braiding point in opposite directions. The deflectors stand in the pathway of the strands from the outside carriers. These deflectors cause the strands from the outer carrier to cross the path of the inner carrier thus interweaving the strands. The interwoven strands then converge to the braiding point to form the braid. The forces on these deflectors are quite high and many strand materials have such resistance to sliding that they cannot be braided on this type machine.

Braiding machines that combine the characteristics of the above two are also known, one being termed a lever arm machine and the other a wheel type machine. The lever arm machine is similar to the rotary machine except that instead of strand deflectors, the strands from the outer carriers are entrained by lever arms. The lever arms are controlled by a cam track which moves the strands of the outer carriers across the paths of the inner carriers.

In the wheel type machine, which is also similar to the rotary machine, the strand material from each outer carrier is entrained by a wheel. The strand material enters the wheel at the center and emerges at some radial distance from the center. The rotation of the wheel moves the strand materials across the pathway of the inner carrier. With such a machine, obtaining a symmetrical braid has been difficult.

EP 0341434 A2 discloses a machine for producing a three dimensional braided article comprising a frame and four carrier arches stacked in a linear manner and attached to the frame. The machine also includes a plurality of moveable carriers and a carrier pushing mechanism. All four of the carrier arches are moveable.

WO2011/119831 A1 discloses a braided, three dimensional scaffold that is useful to augment rotator cuff tendon tissue during healing.

Although many types of braiding machines are known, none of the existing braiding machines is able to produce three dimensional braided matrices with a suitable pore structure for even and random distribution and in-growth of cells for tissue growth and repair.

Thus, there remains a need for a machine and method for producing complex-shaped, three-dimensional engineered fiber matrices that may be used as mechanical support for tissue growth and repair.

There also remains a need for a machine and method for producing a variety of different cross-sectional shapes and sizes in a continuous series on a single machine.

It is an object of the invention to provide an improved machine for making three-dimensional braided matrices, particularly for use as scaffolds for tissue regeneration and surgical meshes.

It is a further object of the invention to provide an improved method of making three-dimensional braided matrices, particularly which can be used to produce a variety of different controlled three-dimensional braided matrices.

It is a further object of the invention to provide improved three-dimensional braided matrices for use as scaffolds for tissue regeneration and surgical meshes.

### SUMMARY OF THE INVENTION

A braiding machine that produces three-dimensional braided matrices with a suitable pore structure for even and random distribution and in-growth of cells for tissue growth and repair is described herein. The braiding machine contains carrier holder arches. The curvature of the arches equalizes the tensions in the yarns, which results in a braided product with a uniform spacing between yarns. The braiding machine also allows for a variety of modifications to the structures of the braided structure, to customize the braided structure for the particular use. For example, the three-dimensional braided matrices may be solid. Alternatively, the resulting matrix may contain breaks (referred to as splits), where one braid becomes two and then rejoins. The split may be along the length or the width of the matrix.

### BRIEF DESCRIPTION OF THE DRAWINGS

Figure 1 is a perspective view of one embodiment of a three-dimensional braiding device, containing two fixed arches and two moveable arches, with only 5 yarns shown for illustration purposes.
Figures 2A and 2B are illustrations of a representative three-dimensional braided matrix. Figure 2B is a magnified partial view of Figure 2A.

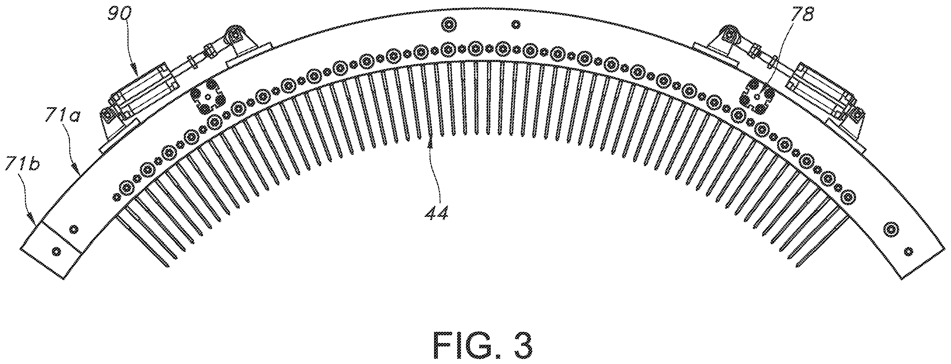

Figure 3 is a front view of the arch portion of the three-dimensional braiding machine.
Figure 4 is an isometric, partial view of the arch portion of the braiding machine, showing the carriers and carrier pushing mechanism.
Figure 5 is a bottom perspective view of the carriers in the rows of slots in the arches of the braiding machine.
Figure 6 is partial bottom isometric view of the braiding machine illustrated in Figure 4.
Figure 7 is an illustration of a representative three-dimensional braided matrix with a split (18) along the machine width.

### DETAILED DESCRIPTION OF THE INVENTION

### I. Braiding Machine

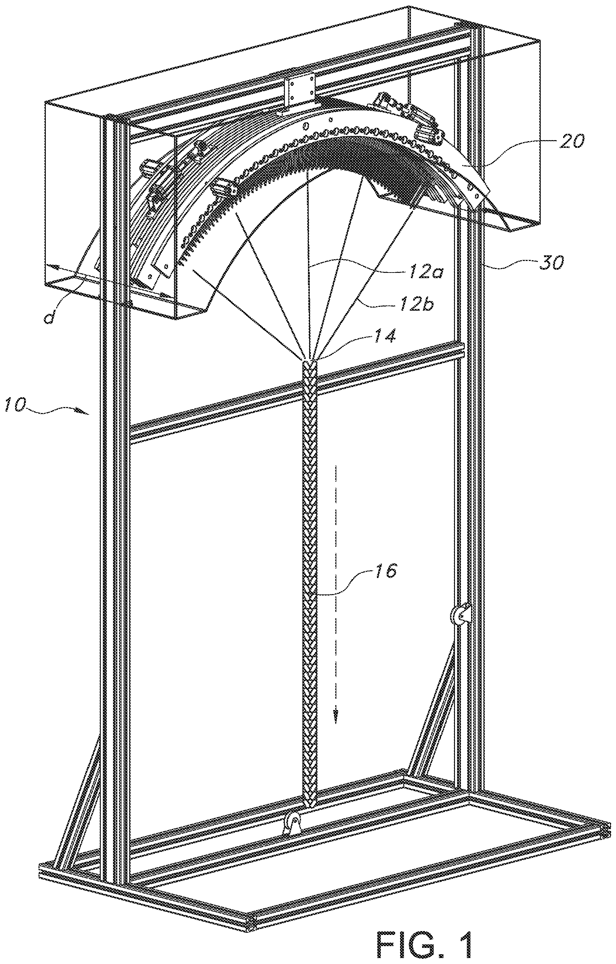

The braiding machine (10) includes at least two moveable braid carrier holder arches (50a, 50b) and at least two stationary braid carrier holder arches (60a, 60b), a plurality of yarn carriers, a plurality of pushers, each of which aligns with a row of carriers, and a frame (30). An exemplary braiding machine is illustrated in Figure 1.

The braid is produced by interlacing of yarns that follow a curve path, established by the curvature of the arches, to equalize the tensions in the yarns. In operation, yarn is supplied from each carrier to a "braid point" (14) where the braid is produced and drawn away. The braid point is located on the centre line of the arc and beyond the sagitta of the arc of the arch. The tension difference is due to the projected length of machine carrier location to the braid point. The speed at which the braid is drawn away or "taken up" may be varied to produce braids of differing structure.

By curving the braiding carrier holders, the length from the tip of the carrier to the braid point is substantially equal for each yarn in the machine (i.e. *l*₁. ≈ *l*₂). This tension control results in a braided product with a uniform spacing between yarns.

The lengths of all of the yarns from the tip of the carrier to the braid point are substantially equal. As used herein "substantially equal in length" means up to ±10% in length, preferably up to ±5%, more preferably ±2%.

The machine preferably braids at least nine fibers or yarns to form the resulting braided matrix. The resulting braid has a number interlaces/interlocks and at least one defined angle relative to the long axis of the support member and forms an elongated braided matrix with a three dimensional braided structure with a uniform spacing between yarns. Uniform yarn spacing is a key to the product quality for 3-D matrices used in implantable medical devices.

### A. Braid Carrier Holder Arches

The braiding machine includes at least two stationary braid carrier holder arches and at least two moveable braid carrier holder arches. The moveable arches are located between the two stationary arches. Optionally, the machine may contain additional moveable arches, such as 3, 4, 5, 6, 7, 8, 9, or 10 movable arches. Increasing the number of moveable arches increases that thickness of the final product.

The arches used in a particular braiding machine have the same arc radius. The arc is a fixed parameter in the machine design and is related to the three-dimensional product produced.

### 1. Arc radius

The arc radius is determined by several factors that include braid angle, yarn size, yarn path, carrier size, and bed plate width. With the fine individual and plied yarns used in biomedical grafts, typically having a range of 50 to 5000 denier yarns, the braided product is relatively small compared to the braiding machine width. With this large ratio (∼27:1), the product can be considered a single point. The arc radius is dominated by the carrier size, carrier spacing, and product braid angle where the minimal difference in yarn path for a typical braiding pattern. A typical arc radius for forming three-dimensional braided matrices for use as biomedical grafts is approximately 80cm. However other radii may be suitable depending on the yarn carrier size, yarn carrier spacing, and product braid angle, such as arc radii ranging from 0.5 m to 1.5 m.

The width of each arch is sufficient to retain each carrier within an arch as it moves from side to side. Typically, the width of the arch is substantially equal to the width of the carrier driver. In a preferred embodiment, each arch is approximately 0.5 inches wide.

### a. Slots and Spacers

### i. Spacers

Each arch contains a plurality of static carriers or spacers and slots (54a 54b), along the length of arch. The spacers (62) are areas along the arch in which no carrier is present. However, a spacer can be converted into or replaced with a static carrier. The static carriers allow for additional yarn to be fed into a braid at a 0° angle (i.e. relatively completely straight of the machine production direction). The static carriers do not move out of their location within a given arch. The fixed or static carriers may be provided in one or more arches, and are preferably in each of the moveable arches. Optionally, the static carriers provide the yarn through hollow axles.

### ii. Slots and Rows of Slots

The slots (54) are regions in the arches in which the carriers may be located and into and through which the carriers slide to reach a slot in another arch. The slots in each of the arches have the same dimensions and can align to form a row of slots, thereby allowing carriers to slide in and out of them as needed.

The number of rows of slots in the moveable arches depends on the width required for the resulting product. A machine used to form a biomedical graft, such as described herein, typically contains at least twenty (20) rows of slots. Suitable ranges include from approximately 20 up to over 100 rows of slots, preferably from about 20 to about 70, more preferably from about 50 to about 65 rows of slots.

Each slot is at an angle towards the center of the arc of the arch. The angle of the carrier (in each slot) is a function of the arch radius. All carriers are in a perpendicular position to the tangent of the arc for the arch. The slots are at the same angle, but whether the angle is positive or negative depends on which side the slot is one relative to the center of the arc.

### B. Carriers

The braiding machine includes a plurality of movable carriers. Each carrier contains a carrier driver (42) and a carrier tensioner (44).

### 1. Yarn Carrier

The yarn carrier size directly affects the machine size and arc radius. The machine size is in linear relation to the carrier size. The carrier has a relatively small diameter, which allows for a high density of yarns in a given area of machine. The diameter of the carrier driver is generally within the standard for the carriers used in braiding machines. For example, the diameter of the carrier driver (42) is typically ranges from 6.35mm to 76.2mm (about 0.25 inches to 3 inches), and preferably is about 12.7mm (about 0.5 inches) diameter. The movable carriers are located along an arch such that there is a minimum distance between each movable yarn carrier and the proximal movably yarn carrier. For example, spacers are preferably less than 50.8mm (about 2 inches) in diameter, more preferably less than 25.4mm (about 1 inch) in diameter, preferably each space is approximately 19mm (about 0.75 inches) in diameter. The diameter of the spacers is equivalent to the spacing between a moving carrier and its proximal moving carrier.

There is no spacing in the machine depth, as carrier drivers are in contact with each other, such that when one carrier driver is pushed, it simultaneously pushes the proximal carrier driver.

### 2. Yarn Tension Control

Braid processes employ multiple yarn tension systems. Yarn tension systems simply couple the yarn to a spring via a carrier system. The braiding machine illustrated in Figure 1 includes a single yarn tension control system. Additionally, yarn storage is possible with more components as is typical of textile braiding machines. Typically yarn tension systems have a relative range of tension, and maximum as carriers move along the machine dimensions.

The curvature of the arches minimizes the carrier path distance to braid point and maintains more accurate and uniform yarn tension while braiding compared to a machine with a straight carrier holder.

A row of slots (80) is formed by a plurality of adjacent slots in adjacent arches that are aligned. Carriers can travel up and down a given row (80) when pushed by a carrier pusher.

Each moveable carrier is arranged for movement up and down adjacent slots (54a, b) in a row (80) when pushed by a carrier pusher and side to side movement via movement in a sideways direction of the moveable arch within which the carrier is located. These motions can be combined, allowing the carrier to transfer from a first arch to a second arch. This changeover motion allows the moveable carriers to move from one row (80), which corresponds with a first carrier pusher, to a second path that corresponds with the slots that align with a second carrier pusher. The second path may be adjacent to the first path, or it may be more distant, *e*.*g*. with two or three or more spacers between with first and second paths.

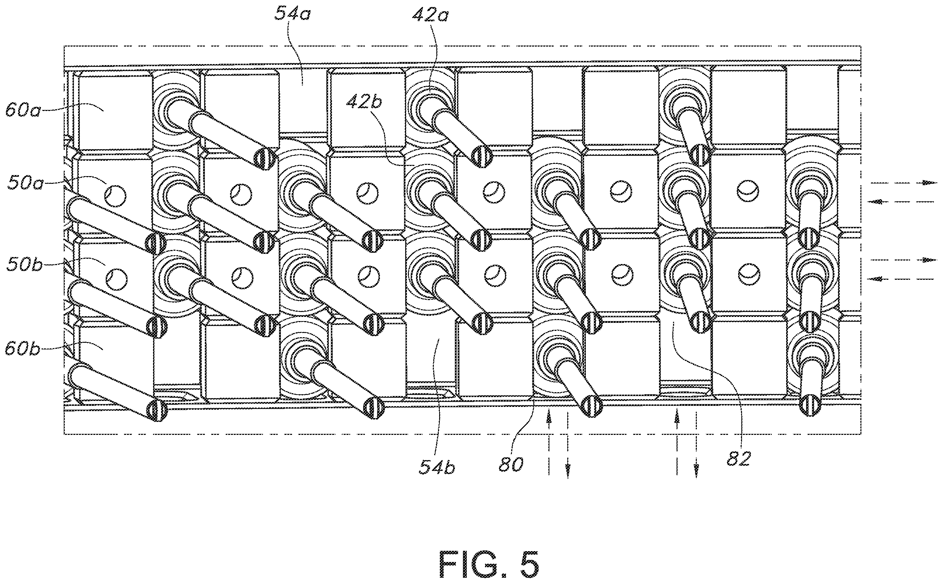

Each carrier acts independently. However, when a carrier pushes a proximal carrier, all of the carriers in the path move to the adjacent slot as a column within the path. *See* Figure 5.

Typically, the carrier also contains a yarn storage means, such as a spool or bobbin (not shown in Figures). The yarn passes through each carrier at a fixed angle relative to the axis of the center of the machine, which corresponds to the apex of the arch. The carriers in each machine feed the yarn at the same angle, which is perpendicular to the tangent of the arc of the arch. For some carriers, this angle is to the left of the center of the machine while for others, the angle is to the right of the center of the machine.

### II. Method of Using the Machine

The braiding machine described herein has three predominant motions to fabricate a braid from a high density of yarn carriers. Two motions are in the arches (side to side) and the carrier push (front to back). The sequence is repeated and the yarn carriers move from front to back while also moving from side to side so that yarn carriers can completely cycle the machine area. The yarns interlace and produce a braid while this occurs.

The third motion is material take down (not shown in Figures) that pulls all braided yarns as it is produced into a three-dimensional product.

The motions are sequenced by a typical computer control system with operator interface to fabricate the braided graph product.

Typically, the machine braids at least nine yarns to form the three-dimensional braided structure. Typically the number of yarns ranges from at least nine up to about 200 yarns. Typical matrices are formed by braiding 30 to 64 yarns. The machine described herein can braid additional yarns, such as up to 134 yarns, or even greater amounts, such as up to 182 yarns. The machine can be scaled up further to allow for greater amounts of yarn.

The number of moveable carriers ranges from at least nine up to about 200, typically from 30 to 64. However, greater number of moveable carriers can be used, such as up to 134 moveable carriers, or even greater amounts, such as up to 182 carriers.

### a. Sizes of Yarn Bundles and Filaments

The size of the yarn bundles (also referred to herein as "yarns") that are braided using the braiding device described herein to form a biomedical graft typically ranges from 50 to 200 denier, preferably from 60 to 100 denier. Individual fibers (filaments) are plied into to create a yarn bundle prior to braiding. Approximately 5 to 50 fibers, preferably from 20 to 40 filaments, are in a yarn bundle. In some embodiments the bundles of fibers are plied in advance. The diameter of an individual filament is from about 15 to about 20 microns. In some embodiments, the fibers have a diameter ranging from about 50 microns to about 150 microns. In particularly useful embodiments, the fiber bundles have a diameter ranging from about 80 microns to about 100 microns.

### A. Motion of the Moveable Arches

The moveable arches are driven by an arch driver (90) with a side to side motion by the machine head. Preferably each arch is controlled by a separate driver. However, alternatively, a driver may control more than one arch, as their motions can be tied in simple braid patterns. Alternate arches move in opposite directions.

The machine head is a stack up of fixed and movable arches that directs the yarn carriers in side to side motions. Coupled with the row movements (track changes), these carriers continue to move side to side until the carrier motion is reversed and the end of the machine.

The machine cycles both arches and carrier pushers alternately and independently for the movement of carrier drivers which in turn move individual yarns. The arches move in opposite directions. The fixed arches serve as a location for the carriers to hold a carrier until next movement cycle. For example, as shown in Figure 5, a carrier is located in a fixed arch (60b). During a cycle, moveable arches (50a and 50b), move in opposite side to side directions, while the fixed arches, and the corresponding carriers located therein, remain in a fixed location.

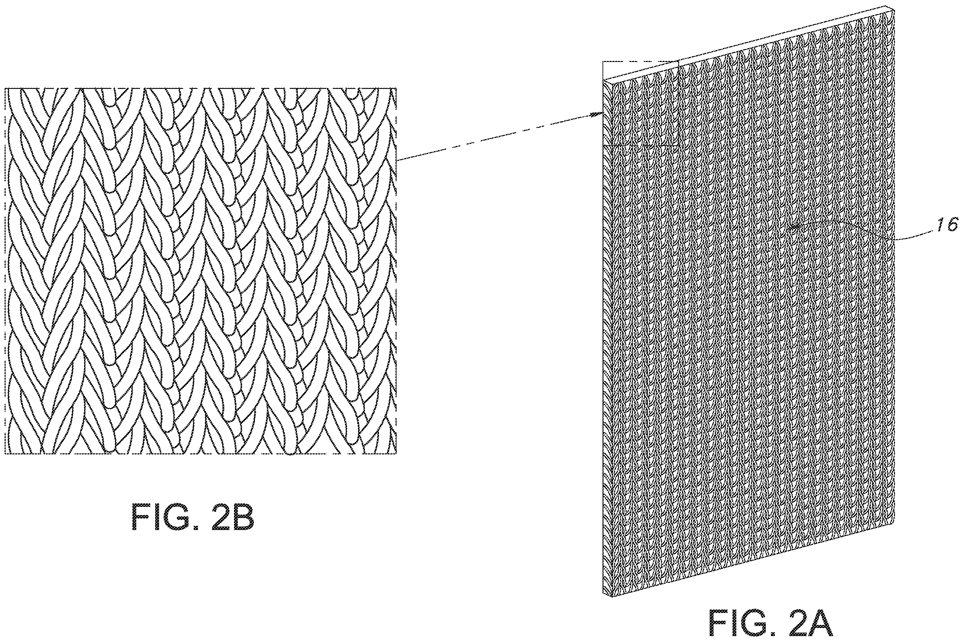

The braiding cycle begins with moving the moveable arches, in opposite directions. This is followed by the moving of carriers by pushing mechanisms (*See* arrows in FIG. 5). The arch are then moved again, followed by the moving of the carriers by the pushing mechanism, and afterwards return to their original position. Yarns are then taken up and the braid point clarified. This completes the braiding cycle to form a solid braid, as illustrated in Figures 2A and 2B.

### B. Carrier Pushing Mechanism

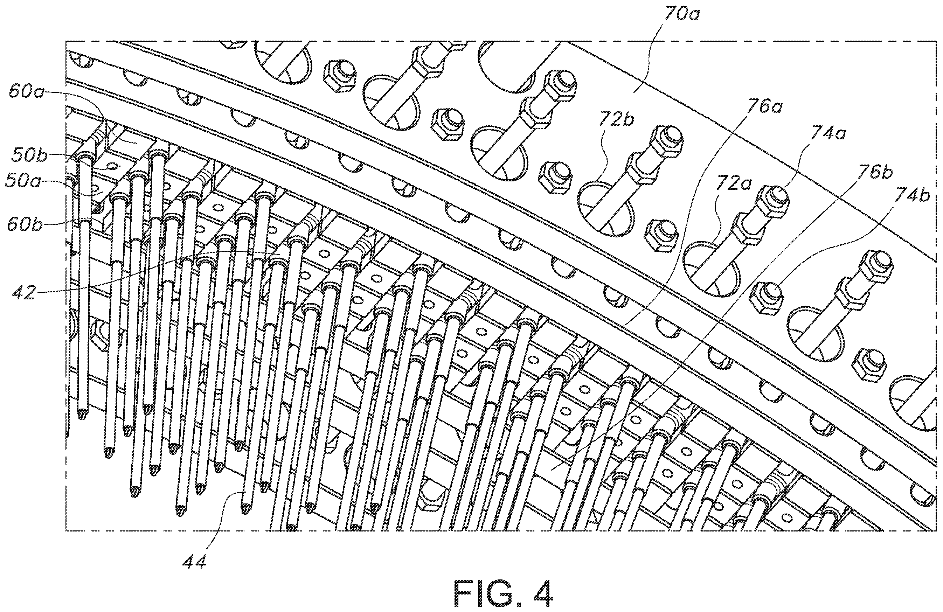

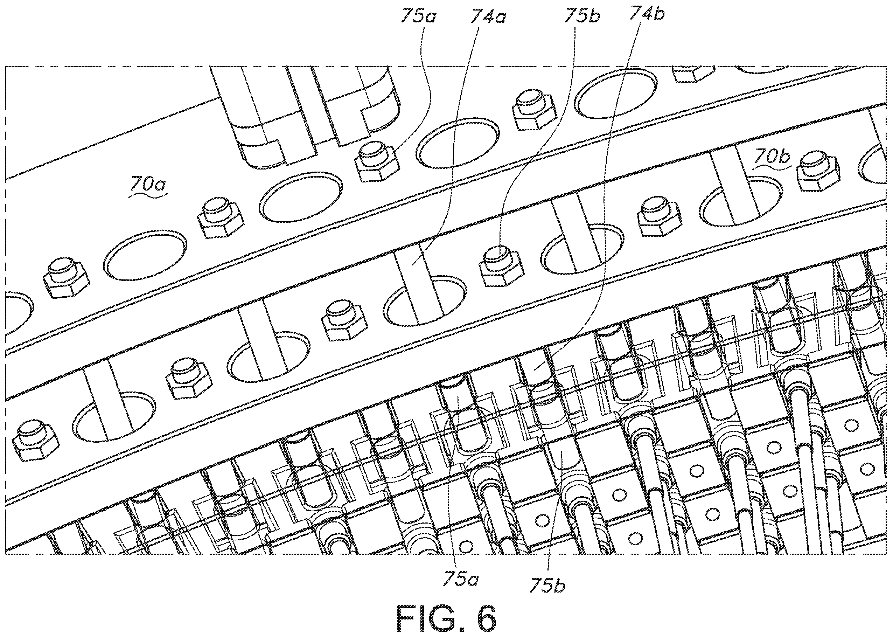

The carrier pushing mechanism controls the movement of the carriers. *See* Figures 4 and 6. It contains carrier push arches (70a, 70b), a plurality of carrier pushers (74a, 74b), and a carrier pusher drive system (78). The carrier pushers are typically in the form of rods, where the distal tip (75a, 75b) of the rod is configured to engage with a carrier driver (42) and push thereby push the driver when the rod is pushed along the depth of the machine.

A pair of carrier push arches (70a, 70b) are located on each side of the plurality of arches, with a pair of push arches located on the outside of each fixed outer arch. The carrier push arches move in and out of the machine, but do not move in the side to side motion.

As shown in Figure 6, in one embodiment, the proximal end of each rod is attached to the carrier push arch. In this embodiment the carrier pushers that are attached to the same carrier push arch are controlled together, such that they all push in the same direction simultaneously.

In an alternative embodiment, the proximal end of each rod is not attached to a carrier push arch. This allows each carrier pusher to be independently controlled.

The rod can be pulled back from the arch; in this configuration, the distal tip (75) of the rod is not in contact with the carrier driver (42), but the rod remains in the opening (72a) in the arch.

The braid can be controlled by the independent control of the carrier push arches (70a, 70b) which move the carrier pushers (74a, 74b). These carrier pushers can be engaged in a unified manner (i.e. all push at the same time). Alternatively, each carrier pusher can be engaged independently of the other carrier pushes with additional independent controlled drivers (78).

A solid three- dimensional braided matrix is formed when the process is continuous with all of the moveable carriers in motion. *See* Figures 2A and 2B.

### C. Braided Graph Split system

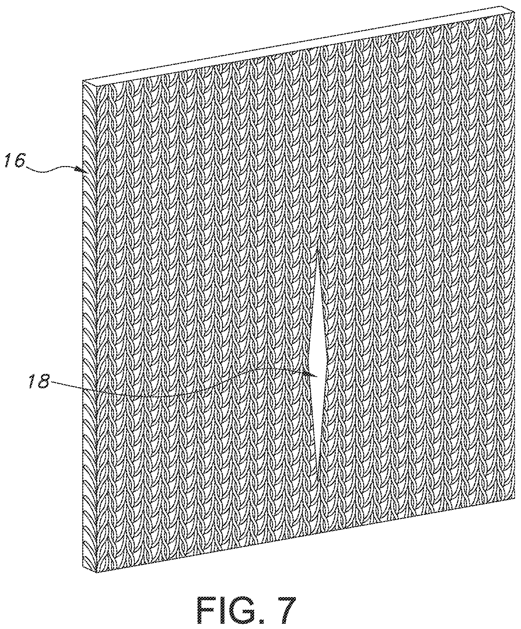

The machine can also create three- dimensional braided matrices that can be bifurcated during the fabrication process to allow for variation of the shape of the devices and customization for application. For example, if the push motion by a carrier pusher is stopped during continuous braiding, then the resulting braid will have a separation point at that location along the machine width. This produces two braids which were previously one unitary braid. Subsequent re-initiation of those carrier push motions will merge the braid back to one unitary structure. An example of a three- dimensional braided matrix (16) with a split (18) along the machine width is illustrated in Figure 7.

The same can be applied to the arch motions when more than two movable arches are employed. Figure 5 shows a portion of an exemplary three-dimensional braiding device with two movable arches, however, these devices could be expanded to have additional moveable arches. If one of the moveable arches does not move, then the machine produces two braids, which were previously one unitary braid that are separate along the machine depth/ thickness direction (*d*). Subsequent re-initiation of those motions will merge the braid back to one unitary structure.

Braid separation is determined as needed for a given product. However, the machine must contain a multiple of arches to form a braided matrix with a split region. For example, with five or more arches moving, the arch(es) in the center can be motionless (fixed for one or more designated cycles), and the braiding can continue on either side of the fixed arch(es).

### III. 3-D Braided Matrices

A polymeric three-dimensional braided matrix that exhibits similar mechanical properties of human fibrous soft tissue may be fabricated using the three-dimensional braiding machine described herein. The mechanical properties of soft tissue and/or the fibrous structures can be determined by the placing a sample in a spring loaded clamp attached to the mechanical testing device and subjecting the sample to constant rate extension (5 mm/min) while measuring load and displacement and recording the resulting strain-stress curve.

In another embodiment, a polymeric three-dimensional braded matrix for repair or augmentation of articular injury may be produced using the machines described herein.

In use, the devices are implanted to match the biomechanical properties of the tissue being repaired. This permits an early return to normal function post-operatively. The implanted device bears applied loads and tissue in-growth commences. The mechanical properties of the biodegradable material of the implant slowly decay following implantation, to permit a gradual transfer of load to the ingrown fibrous tissue. In a preferred embodiment, the degradation of the biodegradable material occurs after about six to twelve months. Additional in-growth continues into the space provided by the biodegradable material of the implant as it is absorbed. This process continues until the biodegradable material is completely absorbed and only the newly formed tissue remains.

The product geometry for the biomedical grafts formed using the machine described herein is typically rectangular or square, for solid, continuous braids (*see, e.g.,* Figures 2A, 2B and 7). Typically the braided matrices produced using the machine described herein are approximately 60 inches long, but can be longer. Then the material is cut, typically with a hot knife to the desired length.

The width and length dimensions of the device can vary within those ranges conventionally used for a specific application. For example, dimensions of about 10 mm by 10 mm to about 100 mm by 100 mm. Typical lengths for the device range from 10 mm to 100 mm. Typical widths for the device range from 10 mm to 100mm. The device can be dimensioned to allow it to be rolled or otherwise folded to fit within a cannula having a small diameter to allow arthroscopic or laparoscopic implantation, fitting within openings on the order of about 0.5 mm to about 30 mm.

The geometric parameters which determine the shape and fiber architecture of three-dimensional braids includes braiding angle distribution, fiber volume fraction, number of carriers, and braiding width. The braiding pattern can depend on braiding machinery/technique used. The scaffold peak load strength range is from 20 to 1000 N, with an initial stiffness range of 20 to 500 N/mm.

The properties of the resulting braided graph can be modified or tailored by addition of yarn carriers in machine width or depth (*d*) direction. This results in a unitary braided graph with specific width, thickness, and length that is suited for the graph as replacement to original medical tissue it replaces. The use of additional moveable arches in the braiding machine produces a thicker braided matrix.

The resulting braided matrix contains an inter-connected, open pore structure that enables even and random distribution and in-growth of cells. The braided structure allows for distribution of mechanical forces over a larger area of tissue at the fixation point(s) compared to woven meshes.

The matrix is a supple, strong, but flexible and more comfortable material that can almost double in size when stretched along the vertical plane, but only extends by about 10% to 20% in length when stretched along the horizontal plane.

### A. Augmentation or Repair of the Breast Tissue or the Abdominal Wall

In one embodiment, the three-dimensional braided matrix may be used to provide mechanical support in a breast reconstruction or mastopexy procedure. In another embodiment, the three-dimensional braided matrix may be used to provide support in hernia repair procedures.

The three-dimensional braided matrix may be formed of multifilament polymeric fibers plied to create yarn bundles. The matrix can be formed of fibers formed from one or more degradable polymers. The degradable matrix is designed to degrade after a period of about six to twelve months following implantation. The matrix will not be completely degraded at this point, rather, the device will degrade to the extent that it loses structural integrity about six to twelve months following implantation. This time period allows the matrix to provide the required structural and flexible mechanical support to support the repair or augmentation of the breast tissue or the abdominal wall, followed by degradation when the support is no longer needed.

In particularly useful embodiments, the braided structure exhibits a stiffness in the range of stiffness exhibited by fibrous soft tissue. Typically, suitable stiffness is in the range of about 10 to about 500 Newtons per millimeter (N/mm), and suitable tensile strength will be in the range of about 20 to about 1000 Newtons (N). In some embodiments, the stiffness of the polymeric fibrous structure will be in the range of about 20 to about 80 N/mm.

The width and length dimensions of the device can vary within those ranges conventionally used for a specific application and delivery device. For example, dimensions of about 10 mm by 10 mm to about 100 mm by 100 mm. The device can be dimensioned to allow it to be rolled or otherwise folded to fit within a cannula having a small diameter to allow arthroscopic or laparoscopic implantation, fitting within openings on the order of about 0.5 mm to about 30 mm. In some embodiments, the fibrous structure defines openings on the order of about 0.5 mm to about 30 mm.

In certain embodiments, the fibrous structure is braided using multifilament PLLA fibers that are plied to create a yarn bundle. Each 60 to 100 denier PLLA fiber is made up of 20 -40 individual filaments. In particularly useful embodiments, the 3-D braided fibrous structure includes about twenty-four 75 denier PLLA fibers made up of 30 individual filaments. The diameter of a 75 denier PLLA fiber is about 80-100 microns, while the diameter of an individual filament is about 15-20 microns. In some embodiments, the fibers have a diameter ranging from about 50 microns to about 150 microns. In particularly useful embodiments, the fibers have a diameter ranging from about 80 microns to about 100 microns.

### B. Repair of Rotator Cuff Tendon Tissue

In one embodiment, the three-dimensional braided matrix may be used to augment the rotator cuff tendon tissue as it proceeds in healing. The braided matrix has two purposes: to provide initial stability to the rotator cuff repair site to allow early mobilization of the upper extremity of the patient, and to allow for reinforcement of rotator cuff tendon repairs to increase the likelihood of successful rotator cuff tendon repairs.

The braided matrix consists of an inter-connected, open pore structure that enables even and random distribution and in-growth of tendon cells. The braided structure allows for distribution of mechanical forces over a larger area of tissue at the fixation point(s). Preferably the porosity of the braided matrix is porosity between 50% and 70% of the volume of the braided matrix. Preferably the pore size ranges from 177µm and 250 µm.

The braided matrix can be formed of a degradable polymer. The degradable material is designed to degrade after a period of about nine to twelve months after affixation to the soft tissue or to bone adjacent to or associated with the soft tissue to be repaired, to allow for repair or augmentation of the tendon prior to the device losing the structural and mechanical support provided by the degradable material.

Preferably, when used for rotator cuff repair, the three-dimensional braded matrix has a peak load strength ranging from 20 to 1300N/cm of width, with an initial stiffness range of 20 to 500 N/mm. Preferably the mesh density ranges from 0.3 to 0.5 bundles/mm. Preferably the suture pullout is greater than 50N. Suture pullout can be measured using a standard test, typically using metal wire for this test.

In preferred embodiments, when used in rotator cuff repair, the three-dimensional braded matrix has relaxed width of between 1 and 8 cm and a tensioned width of between 1 and 6 cm, and a relaxed thickness of between 1.5 mm and 2.5 mm and a tensioned thickness of between 0.8 mm and 1.5 mm.

When produced using the machine described herein, the, braiding angle is typically about 30° +/- 5°.

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize, or be able to ascertain using no more than routine experimentation, many equivalents to the specific embodiments of the invention described herein. Such equivalents are intended to be encompassed by the following claims.

## Claims

1. A machine (10) for producing a three-dimensional braided matrix with controlled porosity comprising
(a) a frame (30),
(b) at least four carrier arches (50a, 50b, 60a, 60b) stacked in a linear manner and attached to the frame,
wherein the two outer arches (60a, 60b) are fixed and the inner arches (50a, 50b) are moveable side to side relative to the frame, and wherein each arch comprises a plurality of slots (54a, 54b), and spacers that alternate along the length of each arch,
(c) a plurality of moveable carriers, wherein each slot (54a, 54b) is configured to receive a carrier, and
(d) a carrier pushing mechanism (70a, 70b, 74a, 74b, 78) configured to push a carrier in a front to back direction relative to the frame, wherein each carrier arch is in the form of an arc, wherein each arc has the same radius,
wherein the carriers are configured to carry yarns that begin forming the braided matrix at a braid point (14),
wherein the braid point is located on the centre line of the arc and beyond the sagitta of the arc.

2. The machine of claim 1, wherein the arches (50a, 50b, 60a, 60b) are aligned such that the slots (54a, 54b) on each arch align with corresponding slots in the proximal arch forming rows of slots.

3. The machine of any one of claims 1 to 2, wherein the lengths from the tip of each carrier to the braid point (14) are within ±10% of each other, preferably within ±5% of each other, more preferably within ±2% of each other.

4. The machine of any one of claims 1 to 3, wherein the total number of arches is 4, 5, 6, 7, 8, 9, or 10 arches.

5. The machine of any one of claims 1 to 4, wherein the carrier pushing mechanism comprises a carrier pusher drive system (78) and a plurality of pushers (74a, 74b), wherein each pusher aligns with one row of slots.

6. The machine of claim 5, wherein the number of pushers (74a, 74b) is equal to the number of rows of slots.

7. The machine of any one of claims 1 to 6, wherein one or more of the spacers comprises a hollow axle configured to receive a yarn.

8. The machine of any one of claims 1 to 7, wherein the slots (54a, 54b) on one side of the midpoint of the arch (50a, 50b, 60a, 60b) are angled toward the center of the arch at a given angle and the slots on the opposite side of the midpoint of the arch are angled toward the center of the arch by the same angle.

9. A method for making three-dimensional braided matrices with controlled shapes and porosity comprising using the machine (10) of any one of claims 1 to 8.

10. The method of claim 9, wherein a braiding cycle begins by moving the moveable arches (50a, 50b) in opposite directions; then the carriers are moved by the carrier pushing mechanisms; and
wherein at the end of the braiding cycle, the yarns are taken up and the braid point is clarified.

## Patentansprüche

1. Maschine (10) zum Erzeugen einer dreidimensionalen verflochtenen Matrix mit geregelter Porosität, Folgendes umfassend:
(a) einen Rahmen (30),
(b) wenigstens vier linear gestapelte und an dem Rahmen befestigte Trägerbögen (50a, 50b, 60a, 60b), wobei die zwei äußeren Bögen (60a, 60b) befestigt sind und die inneren Bögen (50a, 50b) mit Bezug auf den Rahmen seitwärts bewegbar sind und wobei jeder Bogen mehrere Schlitze (54a, 54b) und Distanzstücke umfasst, die sich entlang der Länge eines jeden Bogens abwechseln,
(c) mehrere bewegliche Träger, wobei jeder Schlitz (54a, 54b) konfiguriert ist, einen Träger aufzunehmen, und
(d) einen Trägerdrückmechanismus (70a, 70b, 74a, 74b, 78), konfiguriert, einen Träger in einer Richtung von vorne nach hinten mit Bezug auf den Rahmen zu drücken, wobei jeder Trägerbogen in der Form eines Bogens vorliegt, wobei jeder Bogen denselben Radius aufweist,
wobei die Träger konfiguriert sind, Garnfäden zu tragen, die an einem Verflechtungspunkt (14) beginnen, die verflochtene Matrix auszubilden, wobei der Verflechtungspunkt auf der Mittellinie des Bogens und über den Scheitelpunkt des Bogens hinaus angeordnet ist.

2. Maschine nach Anspruch 1, wobei die Bögen (50a, 50b, 60a, 60b) derart angeordnet sind, dass die Schlitze (54a, 54b) an jedem Bogen an entsprechenden Schlitzen im nächsten Bogen ausgerichtet sind, wodurch Reihen von Schlitzen ausgebildet werden.

3. Maschine nach einem der Ansprüche 1 bis 2, wobei die Längen von der Spitze eines jeden Trägers zum Verflechtungspunkt (14) innerhalb von ±10 % mit Bezug aufeinander, vorzugsweise innerhalb von ±5 % mit Bezug aufeinander, stärker bevorzugt innerhalb von ±2 % mit Bezug aufeinander liegen.

4. Maschine nach einem der Ansprüche 1 bis 3, wobei die Gesamtzahl von Bögen 4, 5, 6, 7, 8, 9 oder 10 Bögen ist.

5. Maschine nach einem der Ansprüche 1 bis 4, wobei der Trägerdrückmechanismus ein Trägerdrückerantriebssystem (78) und mehrere Drücker (74a, 74b) aufweist, wobei jeder Drücker an einer Reihe von Schlitzen ausgerichtet ist.

6. Maschine nach Anspruch 5, wobei die Anzahl der Drücker (74a, 74b) der Anzahl der Reihen von Schlitzen entspricht.

7. Maschine nach einem der Ansprüche 1 bis 6, wobei eines oder mehrere der Distanzstücke eine hohle Achse umfasst, die konfiguriert ist, einen Garnfaden aufzunehmen.

8. Maschine nach einem der Ansprüche 1 bis 7, wobei die Schlitze (54a, 54b) auf einer Seite des Mittelpunkts des Bogens (50a, 50b, 60a, 60b) mit einem bestimmten Winkel zu der Mitte des Bogens hin angewinkelt sind und die Schlitze auf der gegenüberliegenden Seite des Mittelpunkts des Bogens mit demselben Winkel zu der Mitte des Bogens hin angewinkelt sind.

9. Verfahren zum Erzeugen von dreidimensionalen verflochtenen Matrizen mit geregelten Formen und Porosität, umfassend das Verwenden der Maschine (10) nach einem der Ansprüche 1 bis 8.

10. Verfahren nach Anspruch 9, wobei ein Verflechtungszyklus beginnt, indem die beweglichen Bögen (50a, 50b) in einander entgegengesetzte Richtungen bewegt werden; dann werden die Träger durch die Trägerdrückmechanismen bewegt; und wobei am Ende des Verflechtungszyklus die Garnfäden aufgenommen werden und der Verflechtungspunkt bereinigt wird.

## Revendications

1. Machine (10) pour produire une matrice tressée en trois dimensions à porosité régulée comprenant
(a) un cadre (30),
(b) au moins quatre arches de support (50a, 50b, 60a, 60b) empilées de façon linéaire et fixées au cadre,
dans laquelle les deux arches externes (60a, 60b) sont fixes et les arches internes (50a, 50b) sont mobiles côte à côte par rapport au cadre, et dans laquelle chaque arche comprend une pluralité de fentes (54a, 54b) et des espaceurs qui alternent suivant la longueur de chaque arche,
(c) une pluralité de supports mobiles, chaque fente (54a, 54b) étant configurée pour recevoir un support, et
(d) un mécanisme de poussée de support (70a, 70b, 74a, 74b, 78) configuré pour pousser un support dans une direction avant-arrière par rapport au cadre, chaque arche de support ayant la forme d'un arc, chaque arc ayant le même rayon,
dans laquelle les supports sont configurés pour porter des fils qui commencent à former la matrice tressée au niveau d'un point de tresse (14),
dans laquelle le point de tresse est situé sur la ligne centrale de l'arc et au-delà de la flèche de l'arc.

2. Machine selon la revendication 1, dans laquelle les arches (50a, 50b, 60a, 60b) sont alignées de sorte que les fentes (54a, 54b) sur chaque arche s'alignent avec des fentes correspondantes dans l'arche proximale formant des rangées de fentes.

3. Machine selon l'une quelconque des revendications 1 à 2, dans laquelle les longueurs depuis la pointe de chaque support au point de tresse (14) sont à ± 10 % les unes des autres, de préférence à ± 5 % les unes des autres, de manière davantage préférée à ± 2 % les unes des autres.

4. Machine selon l'une quelconque des revendications 1 à 3, dans laquelle le nombre total d'arches est de 4, 5, 6, 7, 8, 9, ou 10 arches.

5. Machine selon l'une quelconque des revendications 1 à 4, dans laquelle le mécanisme de poussée de support comprend un système d'entraînement de pousseur de support (78) et une pluralité de pousseurs (74a, 74b), chaque pousseur s'alignant avec une rangée de fentes.

6. Machine selon la revendication 5, dans laquelle le nombre de pousseurs (74a, 74b) est égal au nombre de rangées de fentes.

7. Machine selon l'une quelconque des revendications 1 à 6, dans laquelle un ou plusieurs des espaceurs comprennent un axe creux configuré pour recevoir un fil.

8. Machine selon l'une quelconque des revendications 1 à 7, dans laquelle les fentes (54a, 54b) sur un côté du point milieu de l'arche (50a, 50b, 60a, 60b) sont inclinées vers le centre de l'arche selon un angle donné et les fentes sur le côté opposé du point milieu de l'arche sont inclinées vers le centre de l'arche selon le même angle.

9. Procédé pour réaliser des matrices tressées en trois dimensions de formes et de porosités régulées comprenant l'utilisation de la machine (10) selon l'une quelconque des revendications 1 à 8.

10. Procédé selon la revendication 9, dans lequel un cycle de tressage commence par le déplacement des arches mobiles (50a, 50b) dans des directions opposées ; puis les supports sont déplacés par les mécanismes de poussée de support ; et
dans lequel, à la fin du cycle de tressage, les fils sont repris et le point de tresse est clarifié.
